# Europäisches Patentamt

## European Patent Office

## Office européen des brevets

(11) Publication number: **0 134 133**
**B1**

(12) **EUROPEAN PATENT SPECIFICATION**

(45) Date of publication of patent specification: **30.07.86**

(21) Application number: **84305419.8**

(22) Date of filing: **09.08.84**

(51) Int. Cl.⁴: **C 07 C 69/54,** C 07 C 67/26,
B 01 J 31/04

(54) Chromium halocarboxylate catalysts for ester synthesis.

(30) Priority: **12.08.83 GB 8321760**

(43) Date of publication of application:
**13.03.85 Bulletin 85/11**

(45) Publication of the grant of the patent:
**30.07.86 Bulletin 86/31**

(84) Designated Contracting States:
**BE DE FR GB IT NL SE**

(56) References cited:
**EP-A-0 001 904**
**GB-A-1 195 485**
**US-A-4 017 429**

(73) Proprietor: **BP Chemicals Limited**
**Belgrave House 76 Buckingham Palace Road**
**London, SW1W 0SU (GB)**

(72) Inventor: **Biggin, Ian Stuart**
**BP Chemicals Limited Barry Sully**
**Penarth Glamorgan Wales, CF6 2YU (GB)**
Inventor: **Dawes, Stephen John**
**BP Chemicals Limited Barry Sully**
**Penarth Glamorgan Wales, CF6 2YU (GB)**

(74) Representative: **Krishnan, Suryanarayana
Kalyana et al**
**c/o The British Petroleum Company plc Patents
Division Chertsey Road
Sunbury-on-Thames Middlesex TW16 7LN (GB)**

Courier Press, Leamington Spa, England.

# 0 134 133

**Description**

The present invention relates to a method of producing esters of alpha, beta-unsaturated carboxylic acids using a chromium halo-carboxylate as an esterification catalyst.

Esters of alpha, beta-unsaturated carboxylic acids are generally produced by reacting the unsaturated acid with an olefin oxide in the presence of a catalyst. The catalyst used is generally a compound of cadmium, manganese, nickel, iron or chromium, compounds of chromium being preferred. For instance, GB 1195485 (BP Chemicals) claims and describes a method for producing such esters using a chromium carboxylate as catalyst. Specifically, the use of chromium-acrylate and -methacrylate as catalysts is disclosed.

It has now been found that the catalyst performance and efficiency of the esterification reaction can be improved by using a halocarboxylate of chromium as the catalyst.

Accordingly, the present invention is a method of producing esters by reacting an alpha, beta-unsaturated carboxylic acid with an epoxide in the presence of a chromium carboxylate as catalyst characterised in that the catalyst is a halocarboxylate of chromium.

The invention is particularly applicable to alpha, beta-unsaturated carboxylic acids, which contain from 3 to 24 carbon atoms. For example, unsaturated monocarboxylic acids such as acrylic acid, methacrylic acid, crotonic acid and linseed fatty acids may be used as starting material. Alternatively, unsaturated polycarboxylic acids such as fumaric acid, maleic acid, itaconic acid and citraconic acid, or the half esters of these carboxylic acids may be used.

The epoxide may be an alkylene oxide derived from a straight chain, branched chain or cyclic olefin, such as, for example, ethylene, propylene, n-butylene, isobutylene, heptylene, cyclohexene or styrene. Epichlorohydrin or glycidyl ethers of monohydric alcohols may also be used.

It is preferred to use a slight molar excess of the epoxide in the reaction mixture. For example, the mole ratio of the epoxide to the alpha, beta-unsaturated carboxylic acid is suitably greater than 1:1. It is preferable to use 1.05 to 1.2 moles of the epoxide per mole of the unsaturated acid.

The halocarboxylates of chromium used as catalyst may be derived from acids which may be saturated monocarboxylic or dicarboxylic acids. The carboxylic acids may be aliphatic, alicyclic or aromatic carboxylic acids. The halocarboxylates suitably contain from 1—10 carbon atoms, preferably from 1 to 4 carbon atoms. Haloacetates are most preferred.

The halogen atom in the halocarboxylate may be flourine, chlorine, bromine or iodine, but is preferably chlorine. The halocarboxylate may contain one, two or three halogen atoms which may be the same or different. Trihalocarboxylates, especially trichlorocarboxylates, are preferred.

The chromium compound used in deriving the halocarboxylate may be a trivalent or a hexavalent chromium compound.

The amount of chromium halocarboxylate used is suitably less than 1 mole per cent based on the the unsaturated carboxylic acid reactant, preferably less than 0.1 mole %, more preferably from 0.02 to 0.1 mole %.

When necessary, a polymerization inhibitor may be employed in the reaction which is any of the polymerisation inhibitors well known in the art. Examples of such inhibitors are hydroquinone, para-methyoxyphenol, nitric oxide and methylene blue. The concentration of inhibitor may be up to 1% by weight. When using nitric acid as inhibitor, the reaction may be carried out in an atmosphere of nitric oxide.

The reaction of the present invention may take place at an elevated temperature, for example 40 to 120°C, preferably from 70—90°C. Temperatures above 120°C may result in excessive polymer formation. Temperatures below 40°C may be employed but at such temperatures the reaction time may be undesirably long. The reaction may be carried out at a pressure above atmospheric, e.g. about 40 psig, (2,75 bar). The process may be operated batchwise or continuously, and in the homogeneous phase or the heterogenous phase. The course of the reaction may be followed by measuring the acidity of the reaction mixture from time to time and hence determining the content of the unreacted unsaturated acid.

A preferred process when using acrylic or methacrylic acid and an epoxide is as follows: an autoclave is charged with the unsaturated acid (containing an inhibitor and catalyst). The autoclave is evacuated and the vacuum broken with nitrogen; the mixture is then heated to a temperature in the range 40 to 120°C. The epoxide is then slowly pumped into the vessel, to give a steady reaction prassure of say 20 to 60 psig, (1,37—4,13 bar). The reaction thus commened is continued at a temperature within the range 40 to 120°C until the acid number of the mixture, determined by the titration of selected portions of the mixture, is at the desired value. Immediately the desired acid number is reached the reaction mixture is crash cooled by indirect heat exchange, for example by contact with water cooled coils. Vacuum is then applied to the autoclave to remove excess epoxide. The 2-hydroxyalkyl acrylate or methacrylate may be removed from the catalyst by distillation.

The process of the present invention is further illustrated by the following Examples.

## Examples

In the examples below the following abbreviations have been used.

HEA — Hydroxyethyl acrylate

HPA — Hydroxypropylacrylate

DPGMA — Dipropylene glycol monoacrylate
HPMA — Hydroxypropyl methacrylate
DPGMMA— Dipropylene glycol monoethacrylate
HEMA — Hydroxyethylmethacrylate
DEGMMA— Diethylene glycol monomethacrylate

## 1. HPA Production using Chromium Halocarboxylates

Acrylic acid (8 moles) inhibited with p-methoxy phenol (700 ppm) and 0.08 mole% of the appropriate chromium carboxylate or halocarboxylate were heated to 80°C. Propylene oxide (8.8 moles) was added over 150 mins., with appropriate cooling to maintain the reaction temperature at 80°C. After completion of oxide addition, the reaction temperature was maintained at 80°C until the concentration of acrylic acid became less than 0.1% wt/wt.

The produced compositions determined by GC analysts for various chromium (halo) carboxylate catalysts are given in Table 1.

TABLE 1

| Example/ Test No. | Catalyst | Composition of Product | |
|---|---|---|---|
| | | HPA | DPGMA |
| C | Chromium III Acrylate | 88.0 | 8.2 |
| 1 | Chromium III Trifluoroacetate | 90.3 | 6.4 |
| 2 | Chromium III Trichloroacetate | 93.3 | 3.4 |
| 3 | Chromium III Tribromoacetate | 90.2 | 6.4 |

C — Comparative Test

## 2. HPMA Production using Chromium Halocarboxylates

Methacrylic acid (8 moles) inhibited with p-methoxy phenol (700 ppm) and 0.08 mole% of the appropriate chromium carboxylate or chromium halo-carboxylate were heated to 80°C. Propylene oxide (8.8 moles) was added over 150 mins, with appropriate cooling to maintain the reaction temperature at 80°C. After the completion of oxide addition the reaction temperature was maintained at 80°C until the concentration of methacrylic acid was less than 0.1 wt/wt.

The product composition, as determined by GC analysis, is given in Table 2.

TABLE 2

| Example No. | Catalyst | Composition of Product | |
|---|---|---|---|
| | | HPMA | DPGMMA |
| 4 | Chromium III Trichloroacetate | 97.5 | 2.2 |
| C | Chromium III Methacrylate | 94.0 | 2.5 |

C — Comparative Test

## 3. HEMA Production using Chromium Halocarboxylates

Methacrylic acid (8 moles) inhibited with p-methoxy phenol (700 ppm) and 0.08 mole% of the appropriate chromium carboxylate or chromium halocarboxylate were charged to an autoclave and heated to 80°C. Ethylene oxide (8.8 moles) was injected over 150 mins. with appropriate cooling to maintain the reaction temperature at 80°C. After the completion of oxide addition, the reaction temperature was maintained at 80°C until the concentration of methacrylic acid was less than 0.1% wt/wt.

The product composition, as determined by GC analysis, is given in Table 3.

# 0 134 133

TABLE 3

| Example No. | Catalyst | Composition of Product | |
|---|---|---|---|
| | | HEMA | DEGMMA |
| 5 | Chromium III Trichloroacetate | 96.2 | 3.1 |
| C | Chromium III Methacrylate | 94.6 | 4.7 |

C — Comparative Test

The above results show that there is a reduction in the amount of dialkylene glycol mono(meth)acrylate formed during production of HPA, HPMA and HEMA compared with normal, unsubstituted chromium carboxylates such as chromium acetate.

4. HEA Production using Chromium Halocarboxylates

Acrylic acid (8 moles) inhibited with p-methoxy phenol (1200 ppm) and 0.08 mole% of the appropriate chromium carboxylate or halocarboxylate were heated to 80°C. Ethylene oxide (8.8 moles) was added over 150 mins. with appropriate cooling to maintain the reaction temperature at 80°C. After completion of oxide addition, the reaction temperature was maintained at 80°C until the concentration of acrylic acid became less than 0.1% wt/wt.

The product composition as determined by GC analysis is given in Table 4.

TABLE 4

| Catalyst | Composition of Product | |
|---|---|---|
| | $\dfrac{HEA}{86.0}$ | $\dfrac{DEGMA}{10.0}$ |
| Cr III acrylate | | |
| Cr III trichloroacetate | 90.0 | 7.5 |

C — Comparative Test

## Claims

1. A method of producing esters by reacting an alpha, beta-unsaturated carboxylic acid with an epoxide in the presence of a chromium carboxylate as catalyst characterised in that the catalyst is a halocarboxylate of chromium.

2. A method according to claim 1 wherein alpha, beta-unsaturated carboxylic acids contains from 3 to 24 carbon atoms.

3. A method according to any one of the preceding claims wherein the alpha, beta-unsaturated carboxylic acid is a monocarboxylic acid selected from acrylic acid, methacrylic acid, crotonic acid and linseed fatty acids, or a polycarboxylic acid selected from fumaric acid, maleic acid, itaconic acid and citraconic acid, or the half esters of these polycarboxylic acids.

4. A method according to any one of the preceding claims wherein the epoxide is epichlorohydrin, a glycidyl ether of a monohydric alcohol or an alkylene oxide derived from a straight chain, branched chain or cyclic olefin.

5. A method according to any one the preceding claims wherein the mole ratio of the epoxide to the alpha, beta-unsaturated carboxylic acid in the reaction mixture is greater than 1:1.

6. A metod according to any one of the preceding claims wherein the halocarboxylates of chromium used as catalyst are derived from halongenated acids selected from a saturated monocarboxylic acid, a saturated dicarboxylic acid and an aromatic carboxylic acid.

7. A method according to claim 6 wherein a halogenated acid is derived from carboxylic acids containing from 1—10 carbon atoms.

8. A method according to any one of the preceding claims wherein the chromium halocarboxylate used is trihalocarboxylate.

9. A metod according to any one of the preceding claims wherein the amount of chromium halocarboxylate used is less than 1 mole per cent based on the unsaturated carboxylic acid reactant.

10. A method according to any one of the preceding claims wherein the reaction is carried out at a temperature from 40 to 120°C.

4

**Patentansprüche**

1. Verfahren zur Herstellung von Estern durch Umsetzung einer alpha-, beta-ungesättigten Carbonsäure mit einem Epoxid in der Gegenwart eines Chromcarboxylats als Katalysator, dadurch gekennzeichnet, daß der Katalysator ein Halogencarboxylat von Chrom ist.

2. Verfahren nach Anspruch 1, worin die alpha-, beta-ungesättigte Carbonsäure zwischen 3 und 24 Kohlenstoffatome enthält.

3. Verfahren nach einem der vorhergehenden Ansprüche, worin die alpha-, beta-ungesättigte Carbonsäure eine Monocarbonsäure, ausgewählt unter Acrylsäure, Methacrylsäure, Crotonsäure und Leinsaman-Fettsäuren oder eine Polycarbonsäure, ausgewählt unter Fumarsäure, Maleinsäure, Itakonsäure und Citraconsäure oder der Halbester dieser Polycarbonsäuren ist.

4. Verfahren nach einem der vorhergehenden Ansprüche, worin das Epoxid Epichlorhydrin, ein Glycidylether eines einwertigen Alkohols oder ein Alkylenoxid, das von einem geradkettigen, verzweigtkettigen oder zyklischen Olefin abstammt, ist.

5. Verfahren nach einem der vorhergehenden Ansprüche, worin das molare Verhältnis des Epoxids zu der alpha-, beta-ungesättigten Carbonsäure in der Reaktionsmischung großer als 1:1 ist.

6. Verfahren nach einem der vorhergehenden Ansprüche, worin die Halogencarboxylate von Chrom, die als Katalysator verwendet werden, von halogenierten Säuren abstammen, die ausgewählt werden unter einer gesättigten Monocarbonsäure, einer gesättigten Dicarbonsäure und einer aromatischen Carbonsäure.

7. Verfahren nach Anspruch 6, worin eine halogenierte Säure abstammt von einer Carbonsäure mit 1—10 Kohlenstoffatomen.

8. Verfahren nach einem der vorhergehenden Ansprüche, worin das verwendete Chrom-Halogencarboxylat ein Trihalogencarboxylat ist.

9. Verfahren nach einem der vorhergehenden Ansprüche, worin die Menge des verwendeten Chrom-Halogencarboxylats weniger als 1 Mol% bezogen auf die ungesättigte Carbonsäure als Reaktionsteilnehmer ist.

10. Verfahren nach einem der vorhergehenden Ansprüche, worin die Umsetzung bei einer Temperatur von 40 bis 120°C durchgeführt wird.

**Revendications**

1. Procédé de production d'esters par la réaction d'un acide carboxylique à insaturation en alpha, bêta, avec un époxyde en présence d'un carboxylate de chrome comme catalyseur, procédé caractérisé en ce que le catalyseur est un halogénocarboxylate de chrome.

2. Procédé selon la revendication 1, dans lequel les acides carboxyliques à insaturation en alpha, bêta contiennent de 3 à 24 atomes de carbone.

3. Procédé selon l'une quelconque des revendications précédentes, dans lequel l'acide carboxylique à insaturation en alpha, bêta est un acide monocarboxylique choisi parmi l'acide acrylique, l'acide méthacrylique, l'acide crotonique et des acides gras de graines de lin, ou bien un acide carboxylique choisi parmi l'acide fumarique, l'acide maléique, l'acide itaconique et l'acide citraconique, ou les hémi-esters de ces acides polycarboxyliques.

4. Procédé selon l'une quelconque des revendications précédentes, dans lequel l'époxyde est l'épichlorhydrine, un éther glycidylique d'un mono-alcool ou un oxyde d'alkylène provenant d'une oléfine linéaire, ramifiée ou cyclique.

5. Procédé selon l'une quelconque des revendications précédentes, dans lequel le rapport molaire de l'époxyde a l'acide carboxylique à insaturation en alpha, bêta, est supérieur à 1:1 dans le mélange réactionnel.

6. Procédé selon l'une quelconque des revendications précédentes, dans lequel les halogénocarboxylates de chrome utilisés à titre de catalyseurs proviennent d'acides halogénés choisi parmiun acide monocarboxylique saturé, un acide dicarboxylique saturé et un acide carboxylique aromatique.

7. Procédé selon la revendication 6, dans lequel un acide halogéné provient d'acides carboxyliques contenant 1 à 10 atomes de carbone.

8. Procédé selon l'une quelconque des revendications précédentes, dans lequel l'halogénocarboxylate de chrome utilisé est un trihalogénocarboxylate de chrome utilisé est trihalogénocarboxylate.

9. Procédé selon l'une quelconque des revendications précédentes, dans lequel la quantité de l'halogénocarboxylate de chrome utilisé est inférieure à 1 mole pour cent, par rapport à l'acide carboxylique insaturé mis en réaction.

10. Procédé selon l'une quelconque des revendications précédentes, dans lequel la réaction est effectuée à une température de 40 à 120°C.